# EUROPEAN PATENT APPLICATION

(11) **EP 4 817 546 A1**
(43) Date of publication of application: **07.10.2026**
(21) Application number: 26170118.9
(22) Date of filing: 01.04.2026
(51) Int. Cl.: A61B 34/10

(54) **SYSTEM AND METHOD OF PLANNING KNEE REPLACEMENT SURGERY ACCOUNTING FOR POST-OPERATIVE PATELLAR KINEMATICS**

(30) Priority: 02.04.2025 BE 202505204
(71) Applicant: Arthro ID, 2600 Berchem (BE)
(72) Inventor: Luyckx, Thomas, 8890 Moorslede (BE); Verstraete, Matthias, 8890 Moorslede (BE); Meyer, Andrew, 8890 Moorslede (BE)
(74) Representative: Willnegger, Eva

(57) **Abstract**

A computer-implemented method of planning knee replacement surgery accounting for postoperative patellar kinematics.

## Description

### FIELD OF THE INVENTION

Total knee arthroplasty (TKA) procedures aim to replace the knee joint using implants and to restore the function of the natural knee joint. Techniques and tools for correctly positioning and orienting femoral and tibial components in TKA are constantly being improved.

Examples include patient-specific instruments (PSI), femoral and tibial custom-made cutting guides, femoral and tibial pre-operative templating tools, and surgical navigation systems.

### BACKGROUND

Figure 8 of WO2022076773A1 to Smith & Nephew teaches a planning method for patellar replacements based on 3D model. Biomechanical measurements of the patellofemoral joint are determined including a mechanical axis and preoperative leg deformity. A 3D model of the patient anatomy is generated based on the input, and the 3D model is characterized in terms of the morphology of the patella. An implant is sized and fitted to the 3D model and implant position and orientation are optimized based on the biomechanics. Results are outputted as a patient report or a surgical plan to a computing device and/or a storage medium.

But this document does not consider the relative position of the patellar orbital axis of motion on the native femur and the femur implant patellar orbital axis as well as the articulating surfaces in view of ensuring that the patella trajectory is not adversely affected by the implant. This document does not account for landmarks at the acetabulum or the torsion of the femur in the planning of the patellar implant / femoral implant position nor does it consider how the patella rests within the trochlear anatomy. Neither does this document consider the impact of the patella through the range of motion on the femoral component.

WO2021149000A2 to Symbios Orthopédie is directed at a method for making of a personalized implant considering the preoperative condition, the prearthritic condition, and the target postoperative HKA alignment to define an individually adapted knee prothesis.

But this document does not disclose considering the relative position of the patellar orbital axis on the femur and the femur implant patellar orbital axis as well as the articulating surfaces in view of ensuring that the patella trajectory is not adversely affected by the implant. WO2023118200A1 pending to Ostesys discloses a planning tool for a knee implant placement based on a 3D model of the tibia and the femur and alignment parameters, such as the hip-knee-ankle angle (HKA), the lateral patellar tile (LPT) and the lateral patellar shift (LPS).

But this document does not disclose considering the relative position of the patellar orbital axis on the femur and the femur implant patellar orbital axis as well as the articulating surfaces in view of ensuring that the patella trajectory is not adversely affected by the implant.

However, despite these efforts, many patients still suffer from short-term and long-term post-operative pain after partial or total knee replacement surgery.

Moreover, currently existing simulations often require dynamic simulations, which require intensive computation and therefore are often perceived as slow.

Therefore, there is still a need for an improved method for supporting total or partial knee replacement arthroplasty. The present invention aims particularly at reducing the risk of patellofemoral complications and at predicting patellar discomfort following total knee arthroplasty. The present invention further aims at predicting the impact of patellar tracking changes following TKA and optimizing total knee component placement to restore healthy knee patellar kinematics.

### SHORT DESCRIPTION OF THE INVENTION

The present inventors found that total or partial knee replacement simulations can be successfully executed with less data computation requirements while approximating the patellar path and thus reducing post-operative discomfort and complications. This is enabled through static simulations of the patellar path. Accordingly, the 3D model does not determine any internal or external forces, stresses and/or pressures. Dynamic simulations, which have high computational requirements are not required.

Accordingly, the present inventors propose a computer-implemented method of planning knee replacement surgery accounting for post-operative patellar kinematics, the method comprising:
▪ Receiving input (imaging data and other data such as the patient file) related to an anatomy of a patient, wherein the input comprises imaging data related to the trochlear groove, optionally the patella, and optionally tibial tuberosity;
▪ Generating a three-dimensional (3D) model of the anatomy based on the input, wherein the 3D model comprises the trochlear groove and optionally the patella, wherein the 3D model compares the trochlear groove of an implant and the native femur optionally correcting the native femur by removing any osteophytes virtually;
▪ Characterizing a morphology of the patellofemoral joint through a plurality of joint poses describing the distal femur relative to the patella based on the 3D model of the femur, the tibia and the patella;
▪ Determining one or more pre-operative morphological parameters based on one or more of the 3D model and the morphology; wherein the pre-operative morphological parameters comprise the patella trajectory and optionally the patella orientation;
▪ Performing one or more morphological and/or biomechanical simulations based on one or more implant-related or morphological parameters, wherein the simulations include one or more poses of the patella-femoral joint through a plurality of tibiofemoral poses;
▪ Optimizing a position, an orientation and optionally a type and size of the femur implant relative to the native femur and patella based on the one or more morphological and/or biomechanical simulations;
▪ Optimizing a position, an orientation and optionally a type and size of the resurfacing of the patella relative to the native femur and patella based on the one or more morphological and/or biomechanical simulations.
▪ Outputting patient-specific planning information to one or more of an external computing device, visual display and a computer-readable storage device; characterized in that
▪ the model uses geometric constraints on the motion of the patella-femoral joint; preferably, preferably the model is only using geometric constraints, even more preferably, the 3D model does not determine any internal or external forces, stresses and/or pressures;
▪ the input comprises one or more imaging data sets, preferably the input comprises one imaging dataset; and/or
▪ the morphological parameters comprise the patella-femoral joint trajectory through a plurality of tibio-femoral joint poses, preferably the trochlear groove through a plurality of tibiofemoral joint poses; and/or
▪ the implant-related parameters include the prosthetic design-related parameters and the prosthetic position and orientation-related parameters; and/or
▪ the patient-specific planning information comprises an optimized position, an orientation and optionally a size of the femur implant, optionally wherein the patient-specific planning information further comprises an optimized resurfacing of the patella.

In another aspect, the morphological parameters include geometric, preferably non-elastic ligament and tendon constraints on the patella, wherein the model comprises geometric, muscle, tendon and ligament models and wherein the patient-specific information includes optimized tibial insert thickness, and implant placement or orientation.

In another aspect, the model includes determining the patellar pose relative to the femur, preferably determined through geometric congruency, and optionally recalculated for a resurfaced patella and optionally wherein the patient specific information includes patellar button placement.

In another aspect, the soft tissue constraints are placed on the congruency pose.

In another aspect, the model comprises contact between the femur and patella to prevent the patella from penetrating the femur surface, wherein a fixed set of tibio-femoral poses is selected and, optionally, wherein at each pose, physiological loads are applied to the patella using muscle and ligament models and wherein the resting point of the patella is determined in function of the trochlear groove through a plurality of flexion orientations and loads.

In another aspect, the patellar trajectory is determined in function of the trochlear groove through a plurality of flexion orientations and optionally loads.

In another aspect, the method comprises a dynamic tibio-femoral movement model and wherein the patellar trajectory is determined through the dynamic movement and wherein the patellar trajectory is used to assess implant location.

In another aspect, the method comprises taking patellar trajectories and assessing the impact of the patellar trajectories on the soft tissue surrounding the knee joint.

In another aspect, the method comprises determining the patella tendon-tibia angles and wherein the patella tendon-tibia angles after knee replacement are optimized to reduce torsion or strain on the soft tissue.

In another aspect, the method comprises assessing the impact of the altered patellar trajectory on the quadriceps muscle.

In another aspect, the method comprises determining the relative angle of the patellar tendon and quadriceps.

In another aspect, the input comprises one or more of X-ray imaging data, MRI imaging data, CT imaging data, ultrasound, and lidar, wherein preferably, the input comprises a 3D imaging data, even more preferably 3D CT imaging data.

In another aspect, the input additionally comprises real time measurements of implant related parameters or morphological parameters or a combination of implant related parameters and morphological parameters before, during, or after surgery, preferably kinematic information related to the hip, tibiofemoral and / or patellofemoral joint.

In another aspect, the patellofemoral position is characterized and optionally visualized by a slice radially directed and defined by a distal mediolateral femoral axis and an angular position around this mediolateral axis defined by the relative position of the patella relative to the femur, preferably wherein the angular position additionally captures the resting position of the patella on the femur, even more preferably only looking at the trochlear groove at this location.

In another aspect, the morphological parameters and / or biomechanical simulations are used to define an optimal position of the implant components simultaneously accounting for the tibiofemoral and the patellofemoral joint, optionally comprising the morphological parameters characterizing the location of the tibia relative to the femur at a plurality of tibiofemoral positions, preferably including the most distal facets of the femoral condyles and / or the most posterior facets of the femoral condyles, wherein optionally the tibiofemoral parameters are correlated with additional morphological and / or simulation parameters characterizing the position of the patella relative to the femur, preferably the radial and mediolateral position of the sulcus of the trochlear groove or the position of the patella relative to the femur, wherein optionally different facets receive a weighting parameter that prioritizes metrics for implant placement preferably where weighting is dependent on local wear status.

A further aspect of the present invention is a system, the system being configured to execute the computer-implemented method of planning knee replacement surgery according to the invention.

### DETAILED DESCRIPTION OF THE INVENTION

The invention is now explained in further detail.

### Definitions

The term "implant" is used to refer to a prosthetic device or structure manufactured to replace or enhance a biological structure, particularly the knee joint.

The term "femur implant" or "femoral component" is used to designate the prosthetic device to replace parts of the distal femur surface in total knee replacement arthroplasty.

The term "pose" is used to describe the position and orientation of a rigid body.

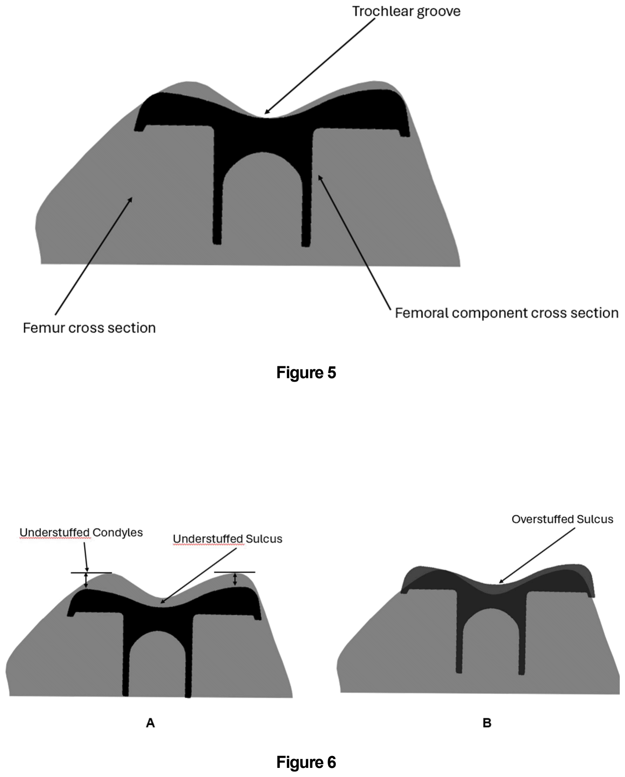

The term "patellar congruency pose" or "congruency pose" describes the patellar pose for a given tibiofemoral joint pose, where the patella is as close as possible to the femur epicondylar axis, femoral component flexion axis, or other anatomical axes or landmarks, while still contacting the trochlear groove and/or the corresponding anterior/distal surface of the femur, and not penetrating the femur surface (Figure 6). The patellar congruency pose naturally is a pose where the shape of the posterior surface of the patella best matches the trochlear groove.

The term "real-time" is used to refer to calculations or operations performed on-the-fly as events occur or input is received by the operable system. However, the use of the term "real-time" is not intended to preclude operations that cause some latency between input and response, so long as the latency is an unintended consequence induced by the performance characteristics of the machine.

The singular forms "a," "an," and "the" include plural references unless the context clearly dictates otherwise. Unless defined otherwise, all technical and scientific terms used herein have the same meanings as commonly understood by one of ordinary skill in the art. Nothing in this disclosure is to be construed as an admission that the embodiments described in this disclosure are not entitled to antedate such disclosure by virtue of prior invention.

As used in this document, the term "comprising" means "including, but not limited to."

### A computer-implemented method of planning knee replacement surgery

The computer implemented method of planning knee replacement surgery applies to both total and partial knee replacement as well as uni-compartmental knee replacement.

### Input

The input relates to an anatomy of a patient and comprises imaging data related to the knee, and optionally the hip, the pelvis and the ankle. The input includes the patella.

In one embodiment, the input comprises reference data of the knee and optionally the hip, the pelvis and the ankle. The simulation of the femur implant position and orientation aims at the reestablishment of the non-pathological knee based on the reference data of the knee and optionally the hip, the pelvis and the ankle.

In one embodiment, the input comprises one or more of X-ray imaging data, MRI imaging data, and CT imaging data. In a preferred embodiment, the input comprises a 3D imaging data, even more preferably 3D CT imaging data.

In one embodiment, the input additionally comprises real time measurements of implant related parameters or morphological parameters or a combination of implant related parameters and morphological parameters during surgery, preferably kinematic information related to the hip, tibiofemoral and / or patellofemoral joint.

### 3D model

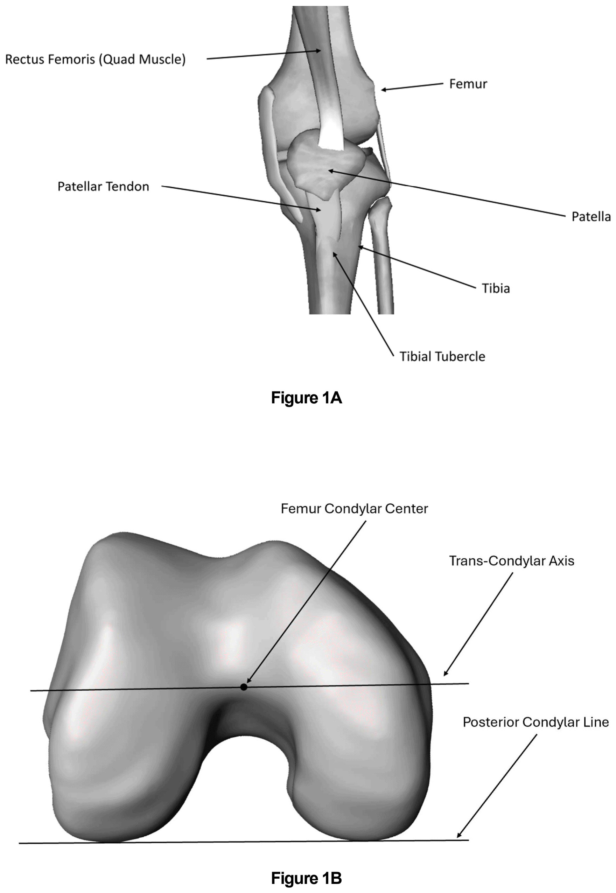

The three-dimensional (3D) model of the anatomy (Figure 1) is based on the input. The 3D model comprises a patella model, a model of the femur, a model of the tibia and optionally a model of the pelvis, the hip, the fibula, the ankle and the bones from the foot. Optionally, the 3D model is corrected for local deformation and wear, for instance by indicating and removing areas with local osteophytes.

### Patellar congruency

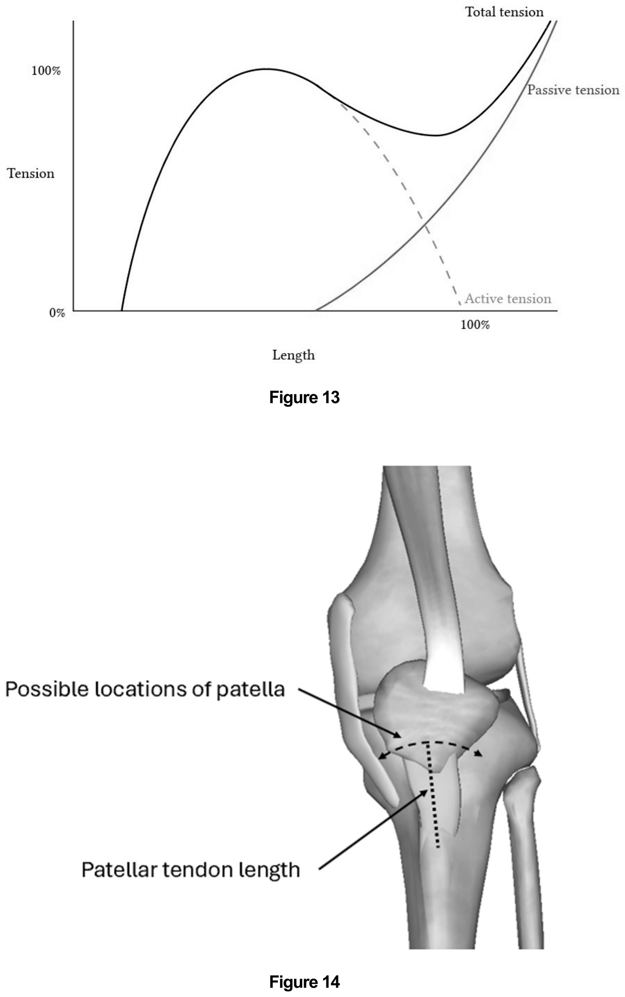

In one embodiment, the solutions for the patellar congruency pose are constrained by soft tissue or other limiting factors (Figure 14).

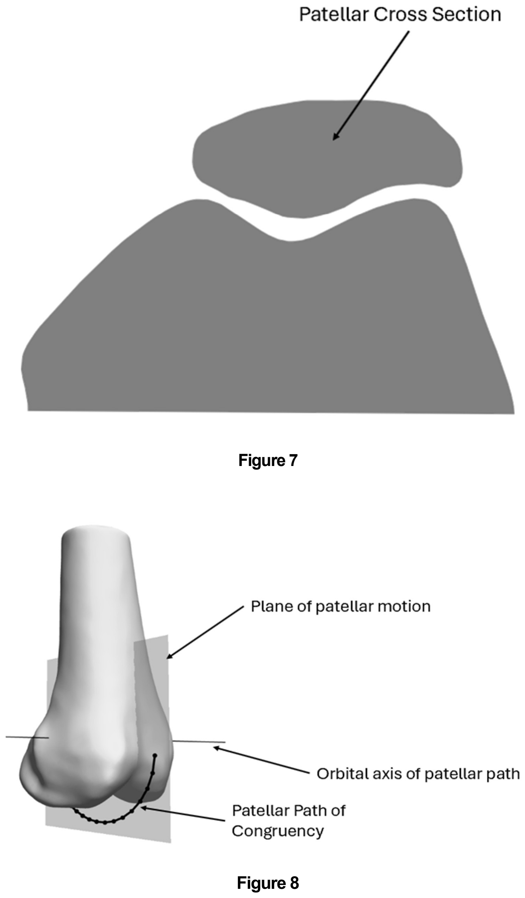

In one embodiment, the positions of congruency for a plurality of tibiofemoral poses is modeled as a line, b-spline, toroid, or other geometric entity (Figure 8).

In one embodiment, the positions of congruency at neighboring tibiofemoral poses are constrained to be similar to each other.

In one embodiment, a margin can be added between the femur and patella representing a soft tissue envelope that the patella cannot penetrate (Figure 7).

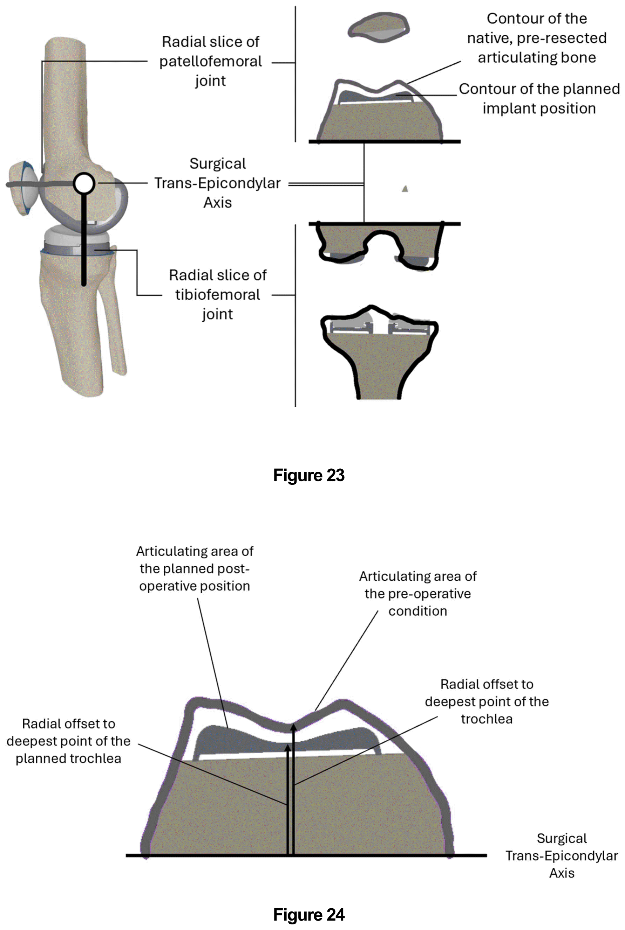

In one embodiment, the computer-implemented method comprises identification of the congruency position, preferably the geometrical constraints and characteristics of both the patella and the trochlear aspect of the femur in order to generate the 3D model of the anatomy of the patellofemoral joint. In a preferred embodiment, an axial cross-section of the patella, as illustrated in Figures 23 to 25, is considered for both the native pre-operative condition and the post-operative condition. In one embodiment, the post-operative condition optionally includes incorporating a patellar resurfacing component. In one embodiment, the computer-implemented method comprises determining the best-fitting position of the patellar cross-section such that:
▪ continuity with adjacent radial slices is ensured by fitting a B-spline, or any other continuous and smooth function, through the centroids of each of said adjacent slices at various angular positions; and
▪ the patellar cross-section matches the femoral trochlear cross-section by minimising the gap distance between both segments, preferably augmented by incorporating a cartilage map of the articulating area of the pre-operative femur and patella and, where no patellar resurfacing is planned, of the post-operative patella ("congruency position").

### Alignment - Comparison of the trochlear groove of an implant and the native femur

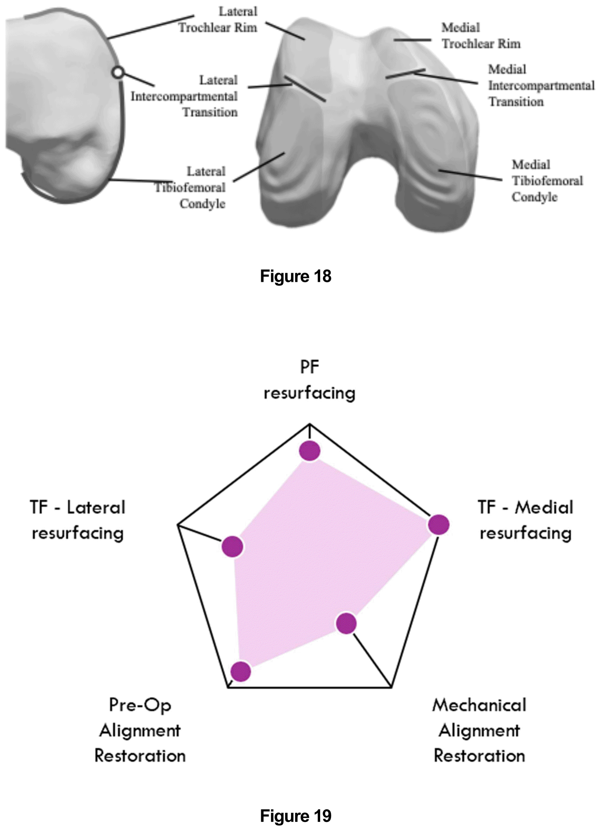

In one embodiment, the computer-implemented method comprises comparing the trochlear groove of an implant and the native femur preferably by considering two morphological aspects to determine alignment (Figures 19 to 25):
▪ a distance metric, and
▪ an angular metric.

In one embodiment, the angular metric informs about the overall or joint specific alignment measures and thereby captures the mechanical alignment variable. In one embodiment, the distance metric considers the joint line offset. In one embodiment, the joint line offset is determined in the axial and sagittal plane. In one embodiment, the joint line offset is determined as the offset in the radial and mediolateral direction. In one embodiment, the radial direction is defined as the distance from a rotation axis (e.g. surgical trans-epicondylar axis) and the articulating surfaces of the knee (implant). In one embodiment, the knee is both mechanically aligned and kinematically aligned at the same time.

In a preferred embodiment, the computer-implemented method comprises considering both the joint line shift in the tibiofemoral joint, and in the patellofemoral joint for assessing the improved as compared to a method without the computer-implemented simulation of the pre-operative condition and of the post-operative condition or optimal implant placement, see Figure 19. In one embodiment, the joint line shifts are determined to be balanced, i.e. a three-compartment alignment or total joint alignment of the components. In one embodiment, all articulating aspects of the knee joint are considered on top of the tibiofemoral joint.

In one embodiment, the computer-implemented method comprises considering each femoral implant position resulting in a given degree of resurfacing for the medial condyle, lateral condyle and trochlea. In one embodiment, the computer-implemented method considers the femoral component resulting in a given Lateral Distal Femoral Angle (LDFA). In one embodiment, the LDFA matches or deviates the pre-operative condition. In one embodiment, the LDFA presents a certain impact on the overall limb alignment. Generally, multiple variables are affected by changing the implant position at once.

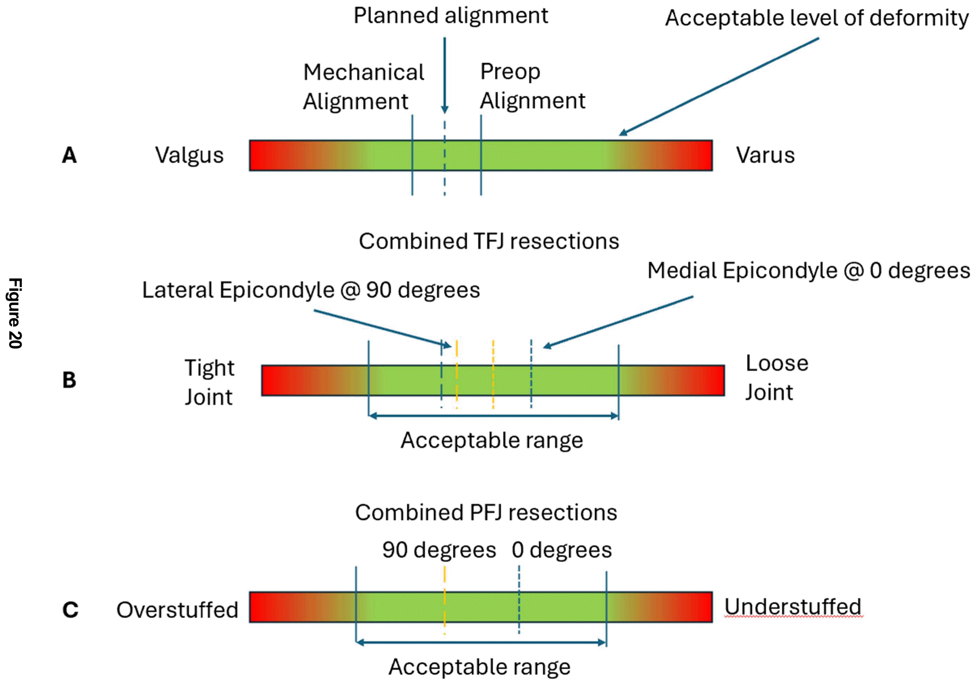

Figure 20 shows the computer-implemented method comprising considering both joint resurfacing of the tibiofemoral (TF) and/or the patellofemoral (PF) joint. In one embodiment, the resurfacing is characterized in that the radial or mediolateral offset between the pre- and post-operative (planned) condition varies through the knee flexion angle. In one embodiment, the knee flexion angle is characterized by a radial slice intersecting with the rotation axis (sTEA). In one embodiment, the knee flexion angle is defined by a normal or by a healthy patient or subject with the mechanical axis of the femur. In one embodiment, offsets in a rotating plan are evaluated at various angles between the normal to the plane and the mechanical axis. In one embodiment, at each angular position, the mean absolute deviation between the planned and pre-operative condition is assessed. In one embodiment, analyzing the absolute deviation between the planned and pre-operative condition through a series of angles, i.e. two or more angles, such as three, four, five, six, seven, eight, nine, ten or more angles, generally results in a mean absolute error. In one embodiment, in case the resurfacing is perfect, the mean absolute error (MAE) equals zero. In one embodiment, the result in values typically ranges from 0 to 10 mm as graphically summarized for example in the radar plot of Figure 19.

In another embodiment, the computer-implemented method of the present invention comprises additionally considering the adjacent components on the patella and tibia. In one embodiment, the pre-operative alignment, preferably the overall limb alignment, are configured to restore the kinematic track of the patella relative to the femur.

In another embodiment, the computer-implemented method comprises considering a projected 2D analysis that looks at the offsets only in one direction. In a preferred embodiment, the computer-implemented method considers the projected 2D analysis in the sagittal plane considering the radial offsets between the pre- and post-operative conditions with the radial offsets being defined as the distances from the axis of rotation (i.e. surgical trans-epicondylar axis) to the most prominent articulating areas. In one embodiment, the distances from the axis of rotation (i.e. surgical trans-epicondylar axis) to the most prominent articulating areas are the maximum distance for a given joint or the apex of a surface (segment).

### Morphology, implant related and morphological parameters

In one embodiment, the 3D model characterizes a morphology of the patella, the femur, the tibia, and optionally the ankle, the fibula, the hip, the pelvis and the bones from the foot such as the calcaneum and the second metatarsal bone.

In one embodiment, the 3D model is used to determine one or more implant-related or morphological parameters based on one or more of the 3D models and the morphology.

In one embodiment, the morphological parameters include local morphological parameters of the knee, and optionally proximal and distal morphological parameters of the ankle, the foot, the hip and the pelvis of a patient.

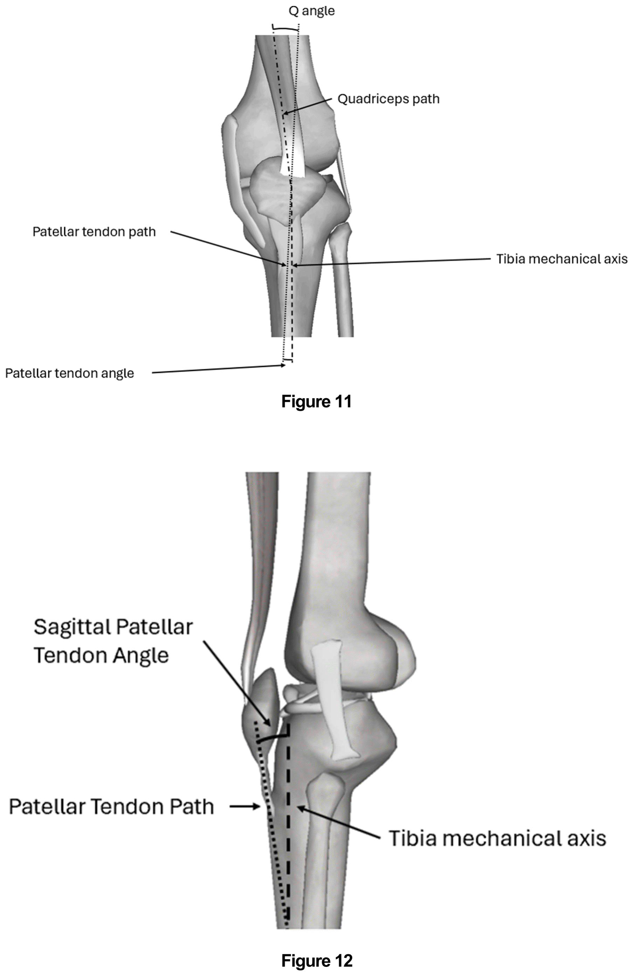

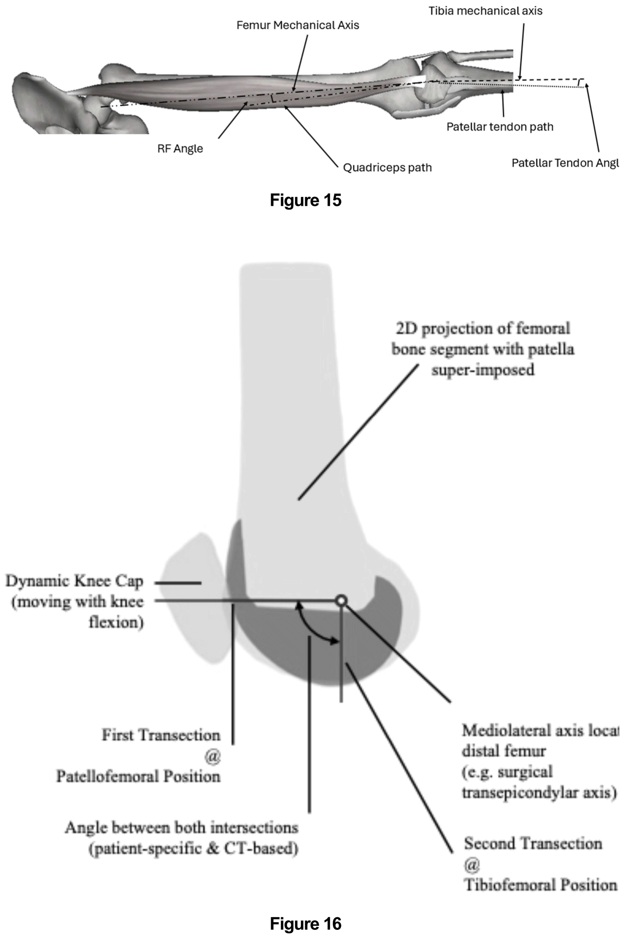

In a preferred embodiment, the proximal anatomical parameters include one or more of the femoral head center, femoral torsion, the acetabular version, the femoral anteversion, the quadriceps tendon alignment (Figure 15), the Q-angle (Figure 11). In a particularly preferred embodiment, the proximal anatomical parameters include at least one, even more preferably at least two, and even more preferably all the proximal anatomical parameters.

In a preferred embodiment, the distal anatomical parameters include one or more of the tibial torsion, the tibial tuberosity to trochlear groove distance projected in the mediolateral and proximo-distal direction. In a particularly preferred embodiment, the distal anatomical parameters include at least one, even more preferably at least two, and even more preferably all the distal anatomical parameters.

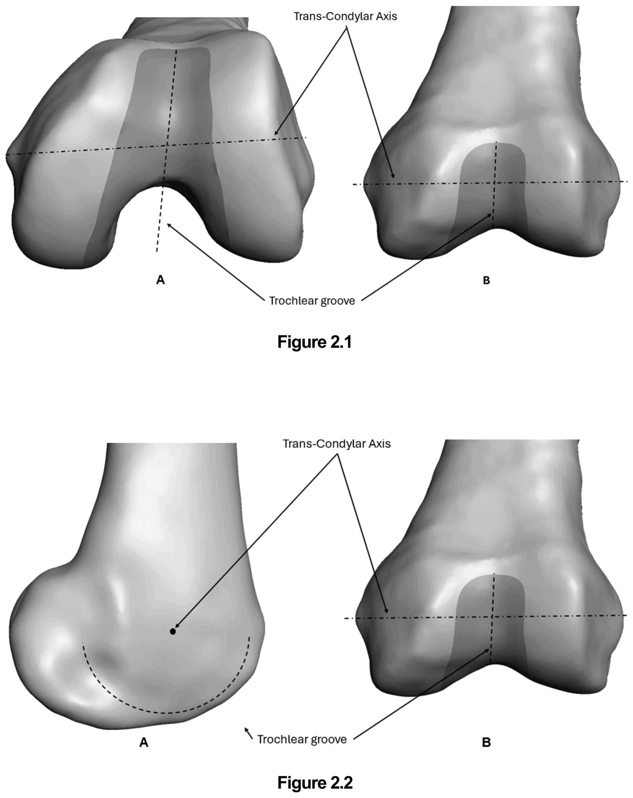

In a preferred embodiment, the local anatomical parameters include one or more of the (surgical) patellar tilt, the trochlear angles (Figure 2), the (surgical) anterior trochlear angle, the lateral trochlear inclination, the sulcus angle, the trochlear depth and width, the patellar trochlear angle, the lateral trochlear rim height, the medial trochlear rim height. In a particularly preferred embodiment, the local anatomical parameters include at least one, even more preferably at least two, and even more preferably all the local anatomical parameters.

The implant-related parameters include the prosthetic design-related parameters and the prosthetic position-related parameters.

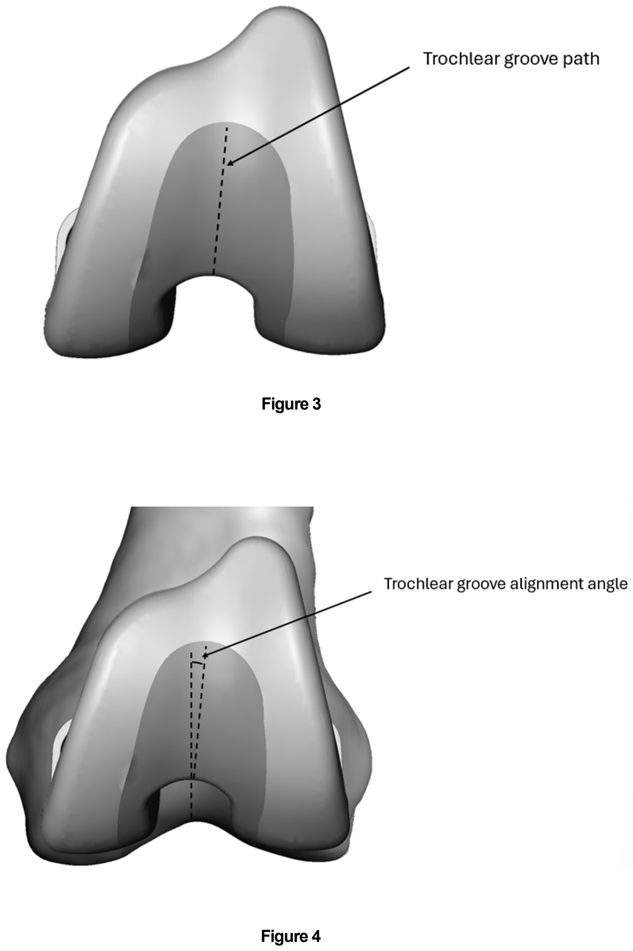

In a preferred embodiment, the prosthetic design related parameters include one or more of the trochlear angles, the sulcus angle and position, the trochlear depth, the trochlear width, the medial trochlear rim height, the lateral trochlear rim height and the anterior trochlear angle (Figure 3). In a particularly preferred embodiment, the prosthetic design related parameters include at least one, even more preferably at least two, and even more preferably all the prosthetic design related parameters.

In a preferred embodiment, the femoral prosthetic position and implant related parameters include one or more of the optimal sizing of the femur, the femur varus/valgus orientation in the coronal plane, the femur intemation/extemal rotation in the axial plane, mediolateral translation in any plane, femoral flexion and the antero-posterior positioning in the sagittal plane, the anterior-posterior sizing in any plane, the proximodistal position in the coronal plane. In a particularly preferred embodiment, the femoral prosthetic position and implant related parameters include at least one, even more preferably at least two, and even more preferably all the prosthetic position and implant related parameters.

In a preferred embodiment, the tibial prosthetic position and implant related parameters include one or more of the tibia medio-lateral position in any plane, the tibial antero-posterior and mediolateral position in the axial plane, the tibial intemal/external rotation in the axial plane, the tibial slope in the sagittal plane, the tibia proximodistal position, the optimal sizing of the tibia implant, the tibial insert thickness and the type of insert (cruciate retaining, cruciate stabilized, medial stabilized, medial pivot, posterior stabilized).

In a particularly preferred embodiment, the tibial prosthetic position and implant related parameters include at least one, even more preferably at least two, and even more preferably all the prosthetic position and implant related parameters.

In a preferred embodiment, the implant related, and the morphological parameters comprise joint surfaces derivable from the 3D model. The joint surfaces include one or more articulating surfaces or one or more non-articulating surfaces or a combination of articulating and non-articulating surfaces.

In a particularly preferred embodiment, the implant related, and the morphological parameters preferably comprise one or more articulating surfaces.

In one embodiment, the implant-related parameters and the morphological further include one or more of:
▪ The sulcus angle, wherein the sulcus angle describes the angle between the medial and lateral facet of the trochlea and the lateral trochlear inclination;
▪ The lateral trochlear inclination, wherein the lateral trochlear inclination describes the angle between the posterior condylar (Figure 1B) line and the line through the lateral facet of the trochlea;
▪ The distal medial femoral articulating surface, the distal lateral femoral articulating surface, the posterior medial femoral articulating surface and / or the posterior lateral femoral articulating surface;
▪ The depth and mediolateral position of the sulcus of the trochlea.

In one embodiment, the parameters further include the patellar tilt and the surgical patellar tilt.

### Biomechanical simulations

The biomechanical simulations are based on the one or more implant-related or morphological parameters. Optionally, the models used to run the simulations have been built after virtually removing osteophytes around the articulating areas of the distal femur, patella and / or tibia. The simulations include one or more poses of the knee or knee implant through a range of motion thereof while optionally accounting for the hip and ankle position during the simulation. The simulations are essential to describe the relative position of various articulating segments relative to each other, especially for estimating post-operative kinematics before surgery. These kinematics could be measured intra-op, but this is also not desired as optical tracking can be challenging in a surgical setting. Indeed, this is particularly relevant for the patellofemoral joint, where attaching optical markers to the bone can be challenging as there is a significant (post-operative) fracture risk associated with this while trackers also take away movement freedom that is essential to complete surgery.

To evaluate the position of the patella in the simulation model, the congruency pose defined above is combined with essential information describing the length of the patellar tendon (Figure 14). This information can be derived from patient-specific medical imaging information such can be obtained from a computed tomography scan, magnetic resonance imaging or the likes. In defining the pose of the patella on the distal femur, the patellar tendon path is defined as described by the attachment sites of the patellar ligament on the proximal tibia (tibial tuberosity) and the patella. This patellar path can be characterized by the sagittal patellar tendon angle through the range of motion (Figure 12). In the coronal plane, this patellar path is characterized by the patellar tendon angle (Figure 11). The load exerted onto the patella by the quadriceps is thereby described by the Q-angle.

### Optimizing a position, orientation and size of the femur implant

The simulations are used to optimize a position, an orientation and size of the femur implant relative to the patella and tibia based on the one or more morphological and/or biomechanical simulations (Figure 5).

In one embodiment, the optimized femur implant position includes adjusting or correcting one or more of the varus, the valgus, internal rotation, external rotation, mediolateral translation in any plane, the femoral flexion, anterior-posterior position, the proximodistal position, the sizing, and the thickness of the planned resection of femur bone and cartilage.

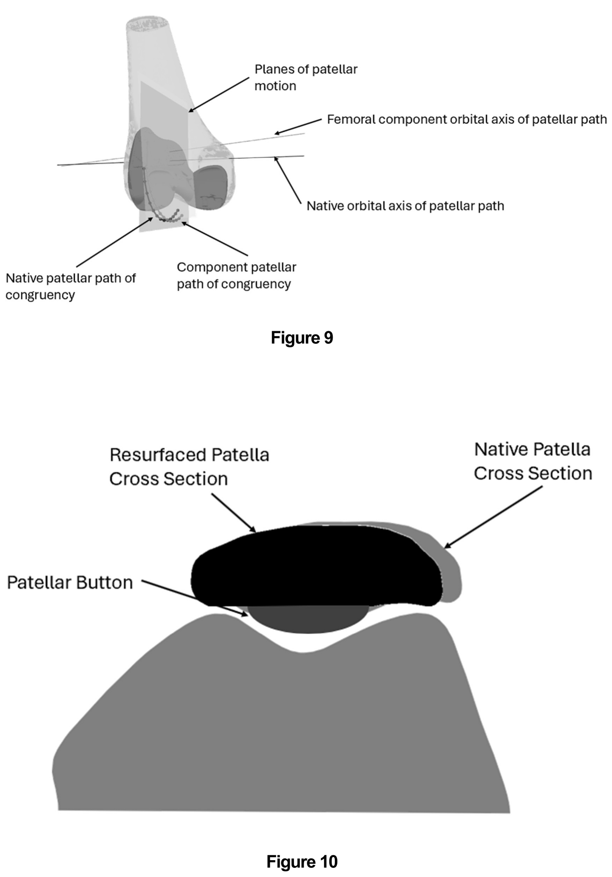

In a preferred embodiment, the optimized orientation ensures a that the patella is not shifted significantly towards or away from the femur distal transcondylar axis (Figure 1B) to avoid post surgical pain and lack of function. These shifts away from the transcondylar axis are referred to as over and understuffing of the patella respectively (Figure 6). Such lack of function can be thought of as repositioning the patella in a position where the muscles linked to the patella such as the quadriceps are not positioned in their functional range along their force-elongation curve (such as shown in Figure 13). Additionally, the orbital axes of the patella can be aligned between the component and native femur anatomy to ensure that the changes in patellar path are constant throughout a plurarlity of knee poses (Figure 9).

In a further preferred embodiment, the optimized femur implant position includes one or more of adjusting or correcting the mediolateral trochlear groove position and orientation (Figure 4), or the medial or lateral trochlear rim height (Figure 18). Optimizing the implant position thereby includes a compromise between various articulating areas, namely the areas of the tibiofemoral and the patellofemoral joint. This optimization is driven by local wear parameters, biomechanical simulations and / or morphological parameters.

### Optimizing a position, orientation and sizing of the tibia implant

In one embodiment, the simulations are used to optimize a position, orientation and size of the tibia implant relative to the patella and femur based on the one or more morphological and/or biomechanical simulations.

In one embodiment, the optimized tibia implant position includes adjusting or correcting one or more of the tibia medio-lateral position in any plane, the tibial antero-posterior and mediolateral position in the axial plane, the tibial intemal/external rotation in the axial plane, the tibial slope in the sagittal plane, the tibia proximodistal position, the optimal sizing of the tibia, the tibial insert thickness and the type of insert (cruciate retaining, cruciate stabilized, medial stabilized, medial pivot, posterior stabilized).

In one preferred embodiment the trochlear groove of the femoral component is aligned with the tibial tuberosity and the antero-posterior axis and rotation of the tibial component.

### Outputting patient-specific planning information

In one embodiment, patient-specific planning information is output to one or more of an external computing device, visual display and a computer-readable storage device.

The patient-specific planning information considers the relative position of the patella to the trochlear groove in function of the flexion of the knee.

In a preferred embodiment, the patient-specific planning information comprises predictive values for one or more of:
▪ The tibio-femoral balance,
▪ The patella-femoral balance,
▪ The size of the femur and tibia and optionally the patella implant components,
▪ Risks for post-operative patella-femoral joint problems,
▪ Prosthetic positions susceptible of causing post-operative patella-femoral joint problems,
▪ Optimized prosthetic position with respect of the patella-femoral joint, the optimized prosthetic position and the optimized orientation of the femur implant relative to the patella through the range of motion of the tibiofemoral joint,
▪ Optimized prosthetic position with respect of the patella-femoral joint, the optimized prosthetic position and the optimized orientation of the tibia implant relative to the patella position through the range of motion of the tibiofemoral joint,
▪ Optimized prosthetic design with respect of the patella-femoral joint, choosing from a database of available designs from different implant manufacturers,
▪ The need for a patellar resurfacing and associated patella implant position (Figure 10) and / or removal of pathological bone segments such as but not limited to osteophytes;
▪ Tibiofemoral vs patellofemoral morphological conflict, and
▪ In case of non-resolution of the tibiofemoral vs patellofemoral morphologic conflict with a standard implant, indicating the requirements for an adapted or custom implant.

### Statistical shape model

In one embodiment, the computer-implemented method comprises using a statistical shape model. In one embodiment, the statistical shape model comprises one or more landmarks defined on the reconstructed distal femur. In one embodiment, the statistical landmarks comprise the medial and lateral epicondyles of the distal femur. In one embodiment, the statistical landmarks additionally comprise statistical shape model to identify characteristic areas on the distal femur. Examples of such characteristic areas include the lateral tibiofemoral condyle, the medial tibiofemoral condyle, the trochlear region, and the medial and/or lateral trochlear rim. Such points or regions may be identified by various means. A well-established approach involves identifying characteristic areas on a generic bone mesh representing the mean shape for a given population. Said mean shape may be derived through statistical analysis of a large cohort, typically comprising 200 or more segmented bone meshes. Such statistical analysis preferably requires all nodes within the mesh to be well-structured and consistently ordered, as is widely described in the literature in the context of defining, developing, and validating statistical shape models. The characteristic areas are subsequently referenced by the indices of the mesh nodes comprised within said areas. Typically, when a newly meshed bone is evaluated, the generic mesh, together with its pre-defined characteristic nodes, is subjected to a series of rigid, affine, and/or elastic transformations so as to fit the newly meshed bone within a pre-defined threshold, typically expressed in millimeters. By this means, the characteristic points of the bone of interest may be identified. An analogous approach may be applied to identify characteristic points or regions on a given implant, once said points have been identified on at least one implant from a family of implants.

With specific reference to the femur and the trochlea, one possible approach consists of identifying a trochlear landmark region within the segmented bone. Similarly, the medial and lateral articulating areas of the tibiofemoral joint may be identified for the patient of interest. Analogously, said characteristic areas may be identified on the proposed implant at any given position, whereby the position may be determined either by the surgeon or through the use of an automated and validated algorithm relying on principles of statistical shape modelling. In addition, the aforementioned statistical shape model-based approach may be employed to identify a first axis on the distal femur. Said axis may be any one of the surgical trans-epicondylar axis (defined by the medial and lateral epicondyle), the cylindrical axis through the articulating facets of the medial and lateral tibiofemoral condyles, or the trans-epicondylar axis. Using said axis, a cylindrical coordinate system may be defined, comprising a radial direction defined perpendicular to said axis, an angular position defined relative to a further bone-fixed axis such as the mechanical axis of the femur, and a mediolateral direction defined along said first axis. Within each of these coordinates, the extremal points, i.e. the nearest and furthest points, within a given area may be determined and characterised as offsets.

More specifically, using the aforementioned coordinate system, the radial offset of the trochlea from the surgical trans-epicondylar axis may be assessed by comparing the radial distance of the deepest point of the trochlea in the pre-operative condition to that of the deepest point of the trochlea at the planned position (on the implant). Said measurements may be compared to one another, reflecting a radial offset between the native and the planned position. Such comparison may be performed at various angular positions within the aforementioned coordinate system, typically ranging between 0 and 120 degrees from the direction perpendicular to the mechanical axis of the femur. By aggregating said radial offsets across this range, a mean absolute difference over the range of motion of the patellofemoral joint may be derived. Analogously, the mediolateral position of the deepest point of the trochlea along the surgical trans-epicondylar axis may be assessed so as to identify a mean absolute mediolateral offset. In a preferred embodiment, said mean absolute difference, or any other statistical descriptor of the distribution between the native and planned trochlear positions, characterises the degree of trochlear resurfacing.

In one embodiment, a similar approach may be applied to each of the medial and lateral tibiofemoral joints, yielding a radial and/or mediolateral offset for the medial and lateral tibiofemoral joint respectively, and subsequently a degree of medial and lateral tibiofemoral resurfacing.

In one embodiment, the aforementioned metrics are more appropriate for defining a planned implant position than the definitions commonly employed in the current scientific literature, such as kinematic, inverse kinematic, or functional alignment. Each of these definitions is characterised by implicit and often ambiguous formulations and, whilst neglecting the patellofemoral aspect, also fails to account for the mediolateral aspect of femoral implant component placement. Accordingly, a given implant position for a given patient may be characterised as a position that achieves a defined degree of patellofemoral, medial tibiofemoral, and lateral tibiofemoral alignment. This characterisation may be considered either as a mean value across the range of motion or at a given flexion angle, flexion being defined either through commonly used definitions of the tibiofemoral joint or, in the absence of the tibia, by the specific angular position relative to the femoral mechanical axis as described above. Offset values between 0 and 10 mm may typically be expected, whereby a mean absolute value of 0 to 2 mm typically indicates a substantially resurfaced joint, and a value of 5 mm or more indicates a large offset between the pre-operative condition and the planned position. Said offsets may in some instance be corrected by up to 2 to 3 mm for cartilage and / or cartilage wear in a given articulating area.

This approach may be extended to further surface-based landmarks, such as the medial and lateral trochlear rim, and may be expanded further still to incorporate the degree to which overall limb alignment - characterised, for instance, by the hip-knee-ankle angle - matches the pre-operative condition or approaches a strict mechanical alignment definition. In one embodiment, these latter two objectives are not necessarily mutually exclusive, as will be appreciated in the case of a patient presenting with a neutral pre-operative overall limb alignment. It is further noted that such alignment definitions may equally be considered at the level of the individual bone - for instance, by reference not to the hip-knee-ankle angle but to the lateral distal femoral angle (LDFA), as is commonly referenced in the art.

In one embodiment the computer-implemented method comprises representing said radial and/or mediolateral offsets using a radial plot aggregating various of the aforementioned metrics, as illustrated in Figure 19. In one embodiment, a large radial distance in this plot thereby represents a high degree of overlap (0 being the max). In one embodiment, a low radial distance represents a low degree of overlap (5 or 10mm) being the central position at the onset of each of medial tibiofemoral, lateral tibiofemoral and patellofemoral axis. In one embodiment, the alignment axes are characterized by a low delta between the pre-operative and planned position reflecting a large radial distance with 0 being the maximum. In one embodiment, a high delta such 5 to 10 degrees is the central most position at the minimum of these axes.

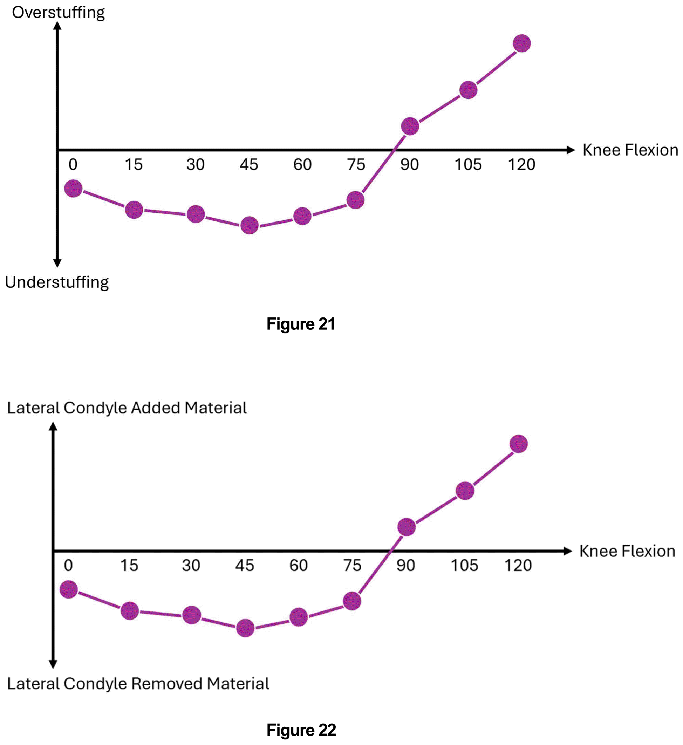

In one embodiment, as illustrated by Figure 21, for an individual area of interest, such as the deepest point of the trochlea, a linear representation of said offset as a function of angular position may further be provided, as illustrated in Figure 21. The example shown in Figure 21 depicts the over- and understuffing of the trochlea at discrete 15-degree intervals.

Figure 23 provides a more practical exemplary visualisation, in which both the tibiofemoral and patellofemoral joints are represented by visualising and analysing radial slices of the respective joints and overlaying their pre-operative and planned positions. In one embodiment, the radial slices constitute the basis for the offsets illustrated in Figures 19 and 21. An example with respect to the trochlea is shown in Figure 24.

In one embodiment, the computer-implemented method relies solely on a model comprising the femoral segment. In a further embodiment, the model may additionally incorporate the patella and tibia, and potentially simplified soft tissue structures connecting said segments. In such an embodiment, the comparison between the pre- and post-operative condition may be driven by the same landmarks. In one embodiment, the computer-implemented method additionally considers landmarks on the counter-surface of the articulating surface or a kinematic track derived from a simulated condition. One such possibility comprises simulating a passive flexion-extension of the knee joint and assessing the change in patellofemoral position at two or more, preferably at each knee flexion angle. Such an approach, relying on kinematic tracks, is more representative in that it additionally accounts for cartilage and other soft tissue artefacts, whilst also considering the deep flexion position at which the femur no longer articulates with the patella solely within the trochlea but also across the bicondylar, distal aspect of the femur.

Where only morphological characteristics are considered, the radial and further offsets for each of the articulating segments may be assessed in isolation. In one embodiment, a combined offset is subsequently defined by summing the trochlear and patellar offsets. This may give rise to a condition in which a positive offset on one surface, such as trochlear overstuffing, is compensated by a negative offset on the other, such as patellar over-resection. The same principle may apply to the tibiofemoral joint. Assessing said offsets in the radial, mediolateral, and angular planes enables a refined and targeted repositioning of the planned femoral implant, allowing the surgeon to adjust the distal and posterior resections so as to guide the radial offset in extension and flexion respectively.

### System

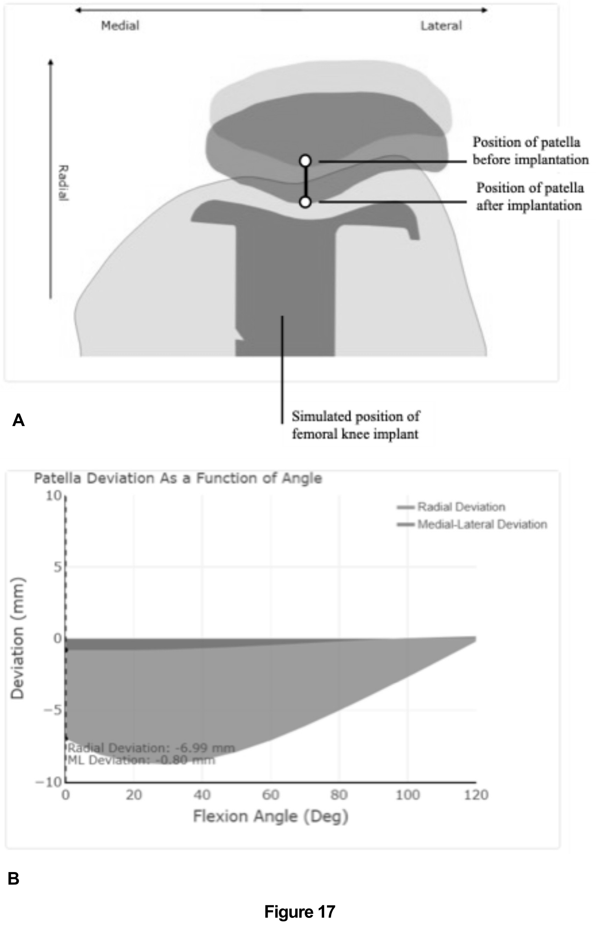

A further aspect of the present invention is a system, the system being configured to execute and display the computer-implemented method of planning knee replacement surgery as disclosed in the invention (Figure 16). The display thereby allows the surgeon to interact with the patient-specific simulations as described and allows the surgeon to virtually optimize the position of the implant to account for the impact of such preferred implant position on the patellofemoral joint and the tibiofemoral joint. The impact is thereby described by an offset between the articulating surface of the intact bone relative to the articulating surface of the artificial joint (implant). This offset can be described by any of a radial and mediolateral distance measure (Figure 17).

### BRIEF DESCRIPTION OF THE DRAWINGS

Further features and advantages will become apparent from the following detailed description, based on appended drawings:
Figure 1A schematically illustrates an embodiment of the method of the present invention including determining the trochlear groove orientation on femur, in particular characteristic points of the femur, the patella, and the tibia, including the tibial tubercle.

In a preferred embodiment, the method also includes determining morphologies of the soft tissues, such as the Recuts femoris (Quad muscle and the patellar tendon. Figure 1 also applies to other femur anatomies.

Figure 1B schematically illustrates an embodiment of the method of the present invention including determining the femur condylar center, the trans-condylar axis and the posterior condylar line. Figure 1b also applies to other femur anatomies.

Figure 2.1 schematically shows an embodiment of the method of the present invention including determining the trochlear groove orientation on femur, in particular the trochlear groove and the trans-condylar axis. Figure 2.1A shows a distal view of the distal femur. Figure 2.1B gives a schematic coronal view of the distal femur. Figure 2.1 also applies to other femur anatomies.

Figure 2.2 schematically shows an embodiment of the method of the present invention including determining the trochlear groove orientation on femur, in particular the trochlear groove and the trans-condylar axis. Figure 2.2A shows a sagittal view of the distal femur. Figure 2.2B gives a schematic coronal view of the distal femur. Figure 2.2 also applies to other femur anatomies.

Figure 3 schematically shows an embodiment of the method of the present invention including determining the trochlear groove path of the implant. Figure 3 also applies to other implant shapes.

Figure 4 schematically shows an embodiment of the method of the present invention including determining the relative trochlear groove alignment and the trochlear groove alignment angle. Figure 4 also applies to other implant shapes and femur anatomies.

Figure 5 schematically shows an embodiment of the method of the present invention including determining the change of shape of the trochlear groove after implantation. Figure 5 also applies to other implant shapes and femur anatomies.

Figure 6 schematically shows an embodiment of the method of the present invention including determining overstuffing and understuffing of the patellar compartment. In overstuffing, the patella is pushed away from the transcondylar axis. In understuffing, the patella is allowed to travel closer to transcondylar axis. Figure 6 also applies to other femur anatomies and implant shapes.

Figure 7 schematically shows an embodiment of the method of the present invention including determining the patella congruency point with the femur. The patella naturally come to rest at specific point on the femur. Figure 7 also applies to other femur and patella anatomies.

Figure 8 schematically shows an embodiment of the method of the present invention including determining the patella path of congruency on femur, associated planes of motions and/or orbital axis. Figure 8 also applies to other femur and patella anatomies.

Figure 9 schematically shows an embodiment of the method of the present invention including comparing the patellar path before and after implant placement. The method preferably includes determining planes of patella motion, the orbital axis of the patellar path of the femur implant and the native orbital axis of the patellar path.

The method preferably includes determining the native patellar path of congruency and the patellar path of congruency of the femur implant. Figure 9 also applies to other femur and patella anatomies.

Figure 10 schematically shows an embodiment of the method of the present invention including determining the patellar resurfacing and its impact on the path of patellar congruency relative to the femur or femoral implant. Figure 10 also applies to other femur and patella anatomies.

Figure 11 schematically shows an embodiment of the method of the present invention including determining the coronal soft tissue angles, preferably the Q angle, the quadriceps path, the patellar tendon path, the tibia mechanical axis, and/or the patellar tendon angle. Figure 11 also applies to other knee anatomies.

Figure 12 schematically shows an embodiment of the method of the present invention including determining the sagittal soft tissue angles, including the sagittal patellar tendon angle, the patellar tendon path and the tibia mechanical axis. Figure 12 also applies to other knee anatomies.

Figure 13 shows the muscle force length relationship.

Figure 14 schematically shows an embodiment of the method of the present invention including determining the limitation of the patellar location by the length of the patellar tendon. Figure 14 also applies to other knee anatomies.

Figure 15 schematically shows an embodiment of the method of the present invention including determining the femur mechanical axis, the RF angle, the quadriceps path, the tibia mechanical axis, the patellar tendon path, and the patellar tendon angle. Figure 15 also applies to other knee anatomies.

Figure 16 schematically shows an embodiment of the method of the present invention including determining radial transections defined by a knee flexion angle, a mediolateral axis such as but not limited to the surgical trans-epicondylar axis and the initial position of the tibia and patella as extracted from a static medical image Figure 16 also applies to other knee anatomies.

Figure 17A schematically shows an embodiment of the method of the present invention including determining or modifying the position of the implant through a translation along three perpendicular axis and a rotation around three perpendicular anatomical axis. Figure 17B shows the patella deviation as a function of the flexion angle. Figure 17 also applies to other knee anatomies.

Figure 18 schematically shows an embodiment of the method of the present invention including determining the delineation between two zones on each of the medial and lateral distal femoral condyles that can be used to evaluate the joint kinematics and the impact of the implant position on the patellofemoral and tibiofemoral joint. Figure 18 also applies to other knee anatomies.

Figure 19 shows an example of an alignment summary for a given joint according to the invention. In this embodiment, alignment numbers range between 0 (meaning the planned alignment matches the theory / pre-op situation) and a large number, for which typically 10 can be thought of as a significant deviation. This considers predominantly the frontal alignment, but parameters in other planes / reference frames can also be considered and potentially aggregated along a given axis of the radial plot by means of taking the mean or mean absolute value through a range of flexion angles (e.g. between 0 and 90 or 0 and 120 degrees of flexion). Each of these axes ranges between a mean absolute error or 0 (maximum, highest value) and 10 (minimum, lowest value). These ranges can be modified, but above numbers represent a reasonable estimate and can be expressed in either mm or degree units for axes referencing offset resp. alignment measures. Additionally, one can overlay whiskers to show the range of the difference across (flexion) angles as to indicate the deviation that is observed with a given, planned implant position through the range of motion.

Figure 20 presents a method of communicating information about the placement an implant with respect to the native geometry and patellar path using line plots. Each line plot is colored, for example from green to red, with one color representing an acceptable value for a metric and the other color representing a value outside clinically expected or surgeon preferred ranges.

The top line plot represents a comparison of the preoperative coronal plane alignment with two post op plans. A first post-op plan references mechanical alignment, where the hip-knee-ankle angle (HKA) is zero. Another line represents a custom plan (indicated by the dotted line) created by a clinician or algorithm that targets a new, planned alignment that is closer to neutral alignment than pre-op but not zero HKA.

The second number line shows combined resection values for the medial and lateral compartment of the tibiofemoral joint at 0 and 90 degrees flexion. If the resections are large, then the joint will be looser as represented by the right side of the number line, and small resections will result in a tighter joint where soft tissues may be stretched. The target zones can additionally be affected by the presence or absence / wear of cartilage in certain zones.

The third number line represents over and understuffing of the patella at multiple flexion angles. If the patella and trochlear resections are too large, the patella will be understuffed, meaning the patellar joint is looser, and if the resections are smaller then the patella will be overstuffed, meaning the soft tissue may be stretched beyond what is experienced in the native knee.

Figure 21 shows the radial offset at exemplary flexion angles for a given joint. In one embodiment, the computer implemented method comprises considering the patellofemoral joint, where either a kinematic and/or a morphological landmark is used to characterize the trajectory of the joint. In one embodiment, the computer-implemented method comprises displaying a combined offset by taking into account the morphological offset from two bone segments constituting a joint, for example:
▪ the radial offset at the level of the patella (when resurfacing the patella obviously), and/or
▪ the radial offset at the level of the femur with its trochlea and articulating intra-condylar area.

Figure 22 shows the concept shown in Figure 21 applied to other metrics, preferably the radial offsets. Combined offsets generally measure if material is added or removed from the joint in a given, i.e. radial, direction at a given flexion angle. Accordingly, in one embodiment, the computer-implemented method comprises considering added/removed material at 0 and 90 degrees flexion, preferably in a continuous version. In one embodiment, the computer-implemented method comprises considering the outer surface of the bone compared to the articulating implant surface in a surgical plan for a given femoral cross section to get a directly correlated value. In one embodiment, these cross sections can be rotated about an axis to calculate these surface deviations at multiple flexion angles. While offsets have a target value, in this case a value of zero would indicate no material is added or removed for a given cross section.

Figure 23 provides a more practical exemplary visualisation, in which both the tibiofemoral and patellofemoral joints are represented by visualising and analysing radial slices of the respective joints and overlaying their pre-operative and planned transsectional areas around an axis that is fixed to the bone, such as but not limited to the surgical trans-epicondylar axes shown in this figure.

Figure 24 shows an example of a visualization and analysis of radial slices with respect to the trochlea for the patellofemoral joint, such as indicated in Figure 23. Shown in the figure are the radial distance from the body-fixed surgical trans-epicondylar axis (sTEA) to the deepest point (sulcus) of the pre-operative condition and the analogous measure on the post-operative condition referencing the planned implant position.

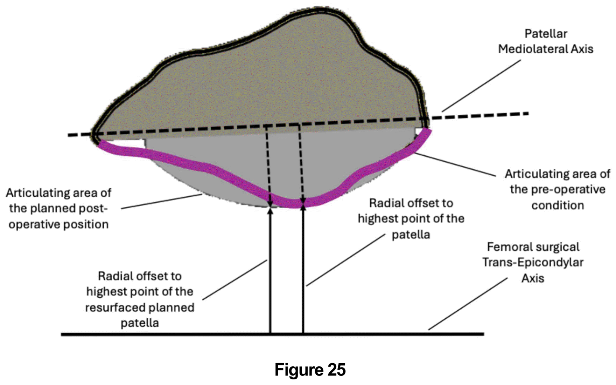

Figure 25 shows the analogous to Figure 24 but for the patella as seen through a radial slice constituting the patellofemoral joint. The figure references the articulating area of the native patella and the articulating area of the planned patellar button. The radial distance to the apex (highest point) of the native and planned position is compared and optionally combined with the radial distance from the trochlear position to assess the overstuffing / understuffing of the patellofemoral joint. The radial distance can thereby be assessed from the body-fixed axis of the femur, and taking into account the kinematic model of the patellofemoral joint. Alternatively, the radial offset can be assessed for the patella in isolation, by considering the radial distance from a body-fixed axis of the patella such as the mediolateral axis of the patella.

### DETAILED DESCRIPTION OF THE DRAWINGS

Figure 1A schematically illustrates an embodiment of the method of the present invention including determining the trochlear groove orientation on femur, in particular characteristic points of the femur, the patella, and the tibia, including the tibial tubercle. In a preferred embodiment, the method also includes determining morphologies of the soft tissues, such as the Rectus femoris, quadriceps muscles and the patellar tendon. Figure 1A also applies to other femur anatomies.
Figure 1B schematically illustrates an embodiment of the method of the present invention including determining the femur condylar center, the trans-condylar axis and the posterior condylar line. Figure 1B also applies to other femur anatomies.
Figure 2.1 schematically shows an embodiment of the method of the present invention including determining the trochlear groove orientation on femur, in particular the trochlear groove and the trans-condylar axis. Figure 2.1A shows a distal view view of the distal femur at a 90°C angle. Figure 2.1B gives a schematic coronal view of the distal femur. Figure 2.1 also applies to other femur anatomies.
Figure 2.2 schematically shows an embodiment of the method of the present invention including determining the trochlear groove orientation on femur, in particular the trochlear groove and the trans-condylar axis. Figure 2.2A shows a sagittal view of the distal femur. Figure 2.2B gives a schematic coronal view of the distal femur. Figure 2.2 also applies to other femur anatomies.
Figure 3 schematically shows an embodiment of the method of the present invention including determining the trochlear groove path of the implant. Figure 3 also applies to other implant shapes.
Figure 4 schematically shows an embodiment of the method of the present invention including determining the relative trochlear groove alignment and the trochlear groove alignment angle. Figure 4 also applies to other implant shapes and femur anatomies.
Figure 5 schematically shows an embodiment of the method of the present invention including the change of shape of the trochlear groove after implantation. Figure 5 also applies to other implant shapes and femur anatomies.
Figure 6 schematically shows an embodiment of the method of the present invention including determining overstuffing and understuffing of the patellar compartment. In overstuffing, the patella is pushed away from the transcondylar axis. In understuffing, the patella is allowed to travel closer to transcondylar axis. Figure 6 also applies to other femur anatomies and implant shapes.
Figure 7 schematically shows an embodiment of the method of the present invention including determining the patella congruency point with the femur. The patella naturally come to rest at specific point on the femur. Figure 7 also applies to other femur and patella anatomies.
Figure 8 schematically shows an embodiment of the method of the present invention including determining the patella path of congruency on femur, associated planes of motions and/or orbital axis. Figure 8 also applies to other femur and patella anatomies.
Figure 9 schematically shows an embodiment of the method of the present invention including comparing the patellar path before and after implant placement. The method preferably includes determining planes of patella motion, the orbital axis of the patellar path of the femur implant and the native orbital axis of the patellar path. The method preferably includes determining the native patellar path of congruency and the patellar path of congruency of the femur implant. Figure 9 also applies to other femur and patella anatomies.
Figure 10 schematically shows an embodiment of the method of the present invention including determining the patellar resurfacing and its impact on the path of patellar congruency relative to the femur or femoral implant. Figure 10 also applies to other femur and patella anatomies.
Figure 11 schematically shows an embodiment of the method of the present invention including determining the coronal soft tissue angles, preferably the Q angle, the quadriceps path, the patellar tendon path, the tibia mechanical axis, and/or the patellar tendon angle. Figure 11 also applies to other knee anatomies.
Figure 12 schematically shows an embodiment of the method of the present invention including determining the sagittal soft tissue angles, including the sagittal patellar tendon angle, the patellar tendon path and the tibia mechanical axis. Figure 12 also applies to other knee anatomies.
Figure 13 shows the muscle force length relationship.
Figure 14 schematically shows an embodiment of the method of the present invention including determining the limitation of the patellar location by the length of the patellar tendon. Figure 14 also applies to other knee anatomies.
Figure 15 schematically shows an embodiment of the method of the present invention including determining the femur mechanical axis, the RF angle, the quadriceps path, the tibia mechanical axis, the patellar tendon path, and the patellar tendon angle. Figure 15 also applies to other knee anatomies.
Figure 16 provides a sagittal view of the distal femur and the femoral implant component indicating two essential radial transections of the distal femur. The radial transections are defined by a knee flexion angle, a mediolateral axis such as but not limited to the surgical trans-epicondylar axis and the initial position of the tibia and patella as extracted from a static medical image such can be thought of as derived from computed tomography or magnetic resonance imaging. The angle between both transections in the sagittal plane is a patient-specific parameter that will depend on the specific position of the patella; this position can vary between a patella baja and a patella alta and is defined amongst others by the length of the patellar tendon. By incorporating this variable angle, the surgeon is able to account of the individual patient's anatomy. The patellofemoral and tibiofemoral joint can be evaluated at various flexion angles by moving the knee flexion through the range of motion, this is accompanied with a changing position of the kneecap and a changing position of both intersections. This allows to visualize the patellofemoral and tibiofemoral joint through various flexion angles; more specifically it allows to evaluate the shift in joint line at all three joints of the knee. Synchronously, the medial and lateral tibiofemoral joint and the patellofemoral joint are visualized for a dynamic range of knee flexion angles.
Figure 17A provides a radial transection of the patellofemoral joint such as defined by the transection line shown in the figure above. Demonstrated is a situation with significant radial offset between the original patellar path and the path resulting from the simulated femoral component. This indicates a significant radial offset, as not only displayed in the radial transactional view but additionally reflected in the x-y graph visualizing the change of the radial patellar position through the range of motion of the knee (knee flexion). At this time, the surgeon is able to modify the position of the implant through a translation along three perpendicular axis and a rotation around three perpendicular anatomical axis. Figure 17B shows the patella deviation as a function of the flexion angle.
Figure 18 demonstrates a series of landmarks that can be used to evaluate the joint kinematics and the impact of the implant position on the patellofemoral and tibiofemoral joint. More specifically, the figure illustrates the delineation between two zones on each of the medial and lateral distal femoral condyles. The delineation is defined by the medial and lateral intercompartmental transition line. This consists of a mediolateral line at either the medial or lateral distal femoral condylar region that defines the difference in articulating area between the patellofemoral and the tibiofemoral joint. The mediolateral axis for each of the condyles can be derived from a simulation model that allows to track the patella through the range of knee flexion. Such simulations can potentially be used in combination with morphological characteristics of the distal femur. Such morphological parameters could be derived from the curvature (of the edge) of the distal femur or the mediolateral position of the apex of the condylar surfaces through a range of radial slices around for instance the surgical trans-epicondylar axis.

## Claims

1. A computer-implemented method of planning knee replacement surgery accounting for post-operative patellar kinematics, the method comprising:
▪ Receiving input (imaging data and other data such as the patient file) related to an anatomy of a patient, wherein the input comprises imaging data related to the trochlear groove, optionally the patella, and optionally tibial tuberosity;
▪ Generating a three-dimensional (3D) model of the anatomy based on the input, wherein the 3D model comprises the trochlear groove and optionally the patella, wherein the 3D model compares the trochlear groove of an implant and the native femur optionally correcting the native femur by removing any osteophytes virtually;
▪ Characterizing a morphology of the patellofemoral joint through a plurality of joint poses describing the distal femur relative to the patella based on the 3D model of the femur, the tibia and the patella;
▪ Determining one or more pre-operative morphological parameters based on one or more of the 3D model and the morphology; wherein the pre-operative morphological parameters comprise the patella trajectory and optionally the patella orientation;
▪ Performing one or more morphological and/or biomechanical simulations based on one or more implant-related or morphological parameters, wherein the simulations include one or more poses of the patella-femoral joint through a plurality of tibiofemoral poses;
▪ Optimizing a position, an orientation and optionally a type and size of the femur implant relative to the native femur and patella based on the one or more morphological and/or biomechanical simulations;
▪ Optimizing a position, an orientation and optionally a type and size of the resurfacing of the patella relative to the native femur and patella based on the one or more morphological and/or biomechanical simulations.
▪ Outputting patient-specific planning information to one or more of an external computing device, visual display and a computer-readable storage device;
**characterized in that**
• the model uses geometric constraints on the motion of the patella-femoral joint; preferably, preferably the model is only using geometric constraints, even more preferably, the 3D model does not determine any internal or external forces, stresses and/or pressures;
▪ the input comprises one or more imaging data sets, preferably the input comprises one imaging dataset; and/or
▪ the morphological parameters comprise the patella-femoral joint trajectory through a plurality of tibio-femoral joint poses, preferably the trochlear groove through a plurality of tibiofemoral joint poses; and/or
▪ the implant-related parameters include the prosthetic design-related parameters and the prosthetic position and orientation-related parameters; and/or
▪ the patient-specific planning information comprises an optimized position, an orientation and optionally a size of the femur implant, optionally wherein the patient-specific planning information further comprises an optimized resurfacing of the patella.

2. The computer-implemented method according to claim 1, wherein the morphological parameters include geometric, preferably non-elastic ligament and tendon constraints on the patella, wherein the model comprises geometric, muscle, tendon and ligament models and wherein the patient-specific information includes optimized tibial insert thickness, and implant placement or orientation.

3. The computer-implemented method of any one of the preceding claims, wherein the model includes determining the patellar pose relative to the femur, preferably determined through geometric congruency, and optionally recalculated for a resurfaced patella and optionally wherein the patient specific information includes patellar button placement.

4. The computer-implemented method of any one of the preceding claims, wherein the soft tissue constraints are placed on the congruency pose.

5. The computer-implemented method of any one of the preceding claims, wherein the model comprises contact between the femur and patella to prevent the patella from penetrating the femur surface, wherein a fixed set of tibio-femoral poses is selected and, optionally, wherein at each pose, physiological loads are applied to the patella using muscle and ligament models and wherein the resting point of the patella is determined in function of the trochlear groove through a plurality of flexion orientations and loads.

6. The computer-implemented method of any one of the preceding claims, wherein the patellar trajectory is determined in function of the trochlear groove through a plurality of flexion orientations and optionally loads.

7. The computer-implemented method of any one of the preceding claims, wherein the method comprises a dynamic tibio-femoral movement model and wherein the patellar trajectory is determined through the dynamic movement and wherein the patellar trajectory is used to assess implant location.

8. The computer-implemented method of any one of the preceding claims, wherein the method comprises taking patellar trajectories and assessing the impact of the patellar trajectories on the soft tissue surrounding the knee joint.

9. The computer-implemented method of any one of the preceding claims, wherein the method comprises determining the patella tendon-tibia angles and wherein the patella tendon-tibia angles after knee replacement are optimized to reduce torsion or strain on the soft tissue.

10. The computer-implemented method of any one of the preceding claims, wherein the method comprises assessing the impact of the altered patellar trajectory on the quadriceps muscle.

11. The computer-implemented method of any one of the preceding claims, wherein the method comprises determining the relative angle of the patellar tendon and quadriceps.

12. The computer-implemented method of any one of the preceding claims, wherein the input comprises one or more of X-ray imaging data, MRI imaging data, CT imaging data, ultrasound, and lidar, wherein preferably, the input comprises a 3D imaging data, even more preferably 3D CT imaging data.

13. The computer-implemented method of any one of the preceding claims, wherein the input additionally comprises real time measurements of implant related parameters or morphological parameters or a combination of implant related parameters and morphological parameters before, during, or after surgery, preferably kinematic information related to the hip, tibiofemoral and / or patellofemoral joint.

14. The computer-implemented method of any of the preceding claims, whereby the patellofemoral position is characterized and optionally visualized by a slice radially directed and defined by a distal mediolateral femoral axis and an angular position around this mediolateral axis defined by the relative position of the patella relative to the femur, preferably wherein the angular position additionally captures the resting position of the patella on the femur, even more preferably only looking at the trochlear groove at this location.

15. The computer-implemented method of any one of the preceding claims, wherein the morphological parameters and / or biomechanical simulations are used to define an optimal position of the implant components simultaneously accounting for the tibiofemoral and the patellofemoral joint, optionally comprising the morphological parameters characterizing the location of the tibia relative to the femur at a plurality of tibiofemoral positions, preferably including the most distal facets of the femoral condyles and / or the most posterior facets of the femoral condyles, wherein optionally the tibiofemoral parameters are correlated with additional morphological and / or simulation parameters characterizing the position of the patella relative to the femur, preferably the radial and mediolateral position of the sulcus of the trochlear groove or the position of the patella relative to the femur, wherein optionally different facets receive a weighting parameter that prioritizes metrics for implant placement preferably where weighting is dependent on local wear status.

16. A system, the system being configured to execute the computer-implemented method of planning knee replacement surgery according to any one of the preceding claims.
